# EUROPEAN PATENT SPECIFICATION

(11) **EP 1 542 700 B1**
(45) Date of publication and mention of the grant of the patent: **09.11.2011**
(21) Application number: 03750546.8
(22) Date of filing: 15.09.2003
(51) Int. Cl.: A61K 31/675, A61P 19/00, A61P 19/10

(54) **ZOLEDRONIC ACID FOR PREVENTING OR REDUCING SECONDARY FRACTURES AFTER HIP FRACTURE**
ZOLEDRONSÄURE ZUR PRÄVENTION ODER VERMINDERUNG VON SEKUNDÄRFRAKTUREN NACH HÜFTFRAKTUR
L'ACIDE ZOLEDRONIQUE POUR LA PREVENTION OU LA REDUCTION DE FRACTURES SECONDAIRES CONSECUTIVES A UNE FRACTURE DE LA HANCHE

(30) Priority: 16.09.2002 US 411067 P
(43) Date of publication of application: 22.06.2005
(73) Proprietor: Novartis AG, 4056 Basel (CH); Novartis Pharma GmbH, 1230 Wien (AT); Lyles, Kenneth W., Durham, NC 27707 (US)
(72) Inventor: LYLES, Kenneth, W., Durham, NC 27707 (US); HOROWITZ, Zebulun, David, Basking Ridge, NJ 07920 (US)
(74) Representative: Leon, Susanna Iris
(86) International application number: PCT/EP2003/010239
(87) International publication number: WO 2004/024166

(56) References cited:
- US-A- 5 965 547
- US-B1- 6 255 288
- REID IAN R ET AL: "Intravenous zoledronic acid in postmenopausal women with low bone mineral density" NEW ENGLAND JOURNAL OF MEDICINE, vol. 346, no. 9, 28 February 2002 (2002-02-28), pages 653-661, XP009021019 ISSN: 0028-4793
- SOLOMON C G: "Bisphosphonates and osteoporosis" NEW ENGLAND JOURNAL OF MEDICINE 28 FEB 2002 UNITED STATES, vol. 346, no. 9, 28 February 2002 (2002-02-28), page 642 XP009021021 ISSN: 0028-4793
- NEED ALLAN G ET AL: "Vitamin D status: Effects on parathyroid hormone and 1,25-dihydroxyvitamin D in postmenopausal women" AMERICAN JOURNAL OF CLINICAL NUTRITION, vol. 71, no. 6, June 2000 (2000-06), pages 1577-1581, XP002261420 ISSN: 0002-9165
- LYLES, KENNETH W. ET AL: "Zoledronic Acid and Clinical Fractures and Mortality after Hip Fractures", NEW ENGLAND JOURNAL OF MEDICINE, vol. 357, no. 18, 1 November 2007 (2007-11-01), pages 1799-1809,

## Description

### Background of the Invention

### Field of the Invention

This invention relates to new therapeutic uses of zoledronic acid or salts or hydrates thereof, according to the claims.

### Description of the Related Art

Bisphosphonates are analogues of pyrophosphate and exhibit marked effects on bone metabolism. The bisphosphonates' characteristic phosphorus-carbon-phosphorus bond (P-C-P) renders the class resistant to hydrolysis by phosphatases and enables these molecules to bind tightly to calcified bone matrix. They are very effective inhibitors of osteoclastic bone resorption and have been used clinically in Paget's disease of bone, osteoporosis, hypercalcemia of malignancy and bone metastases. Zoledronic acid, i.e.1-hydroxy-2-(imidazol-1-yl)ethane-1,1-diphosphonic acid, is a nitrogen containing bisphosphonate (third generation). In a variety of assays of bone containing metabolism, zoledronic acid has demonstrated inhibition of bone resorption *in vitro* at concentrations of 0.3-30 nM, and *in vivo* at doses of 0.3-30 µg/kg without exerting any untoward effects on either bone formation or mineralization.

Hip fractures are the most devastating of the osteoporotic fractures. Patients with hip fractures lose bone mass and muscle mass in the year following the fracture. There is a very high risk of subsequent fractures after hip fracture in both men and women. These secondary fractures significantly impact the quality of life of patients already struggling to recover from their initial hip fracture. Men and women hip fracture patients have much to gain from the development of an effective secondary fracture prevention intervention.

It has now been found that administration of a zoledronic acid or salts or hydrates thereof to a patient who has recently undergone surgical repair of a hip fracture, significantly reduces the rate of all subsequent osteoporotic skeletal fractures.

### Summary of the Invention

Accordingly, the present invention provides the use of zoledronic acid or a pharmaceutically acceptable salt or hydrate thereof in the preparation of a medicament for the treatment of osteoporosis post hip fracture characterised in that the treatment is to prevent or reduce subsequent osteoporotic skeletal fractures in a patient in need of such treatment, said patient having undergone repair of a hip fracture within the past 90 days, and wherein the medicament is administered once per year.

The present invention is particularly applicable to the patients who have had a hip fracture repair within the past three months, e.g., the past 90 days, past 60 days, the past 5-45 days, the past 1-42 days, past 7-42 days or past 1-7 days, preferably wherein the hip fracture repair was within the past 90 days.

The use of the present invention represents an improvement to existing therapy of patients who have undergone a hip fracture in which zoledronic acid is used to prevent or curtail the occurrence of any subsequent osteoporotic skeletal fractures.

### Detailed Description of the Invention

Thus in the present description the terms "treatment" or "treat" refer to preventative treatment.

Zoledronic acid, as used herein, is intended to include the free acid itself, i.e., 1-hydroxy-2-(imidazol-1-yl)ethane-1,1-diphosphonic acid, as well as any pharmaceutically acceptable salts and hydrates thereof and solvates thereof forming from other solvents used for its crystallization.

1-hydroxy-2-(imidazol-1-yl)ethane-1,1-diphosphonic acid and its pharmacologically acceptable salts, hydrates and solvates are well-known from the literature. They can be prepared by procedures known in the art, such as described, e.g., in U.S. Patent No. 4,939,130. See also U.S. Patent Nos. 4,777,163 and 4,687,767.

Pharmacologically acceptable salts are preferably salts with bases, conveniently metal salts derived from Groups Ia, Ib, IIa and IIb of the Periodic Table of the Elements, including alkali metal salts, e.g., potassium and especially sodium salts, or alkaline earth metal salts, preferably calcium or magnesium salts, and also ammonium salts with ammonia or organic amines.

Especially preferred pharmaceutically acceptable salts are those where one, two, three or four, in particular one or two, of the acidic hydrogens of the zoledronic acid are replaced by a pharmaceutically acceptable cation, in particular sodium, potassium or ammonium, in the first instance sodium.

A very preferred group of pharmaceutically acceptable salts is characterized by having one acidic hydrogen and one pharmaceutically acceptable cation, especially sodium, in each of the phosphonic acid groups.

Zoledronic acid is preferably used in the form of pharmaceutical compositions that contain a therapeutically effective amount of zoledronic acid active ingredient optionally together with or in admixture with inorganic or organic, solid or liquid, pharmaceutically acceptable carriers which are suitable for administration.

The pharmaceutical compositions may be, for example, compositions for enteral, such as oral, rectal, aerosol inhalation or nasal administration, compositions for parenteral, such as intravenous or subcutaneous administration, or compositions for transdermal administration, e.g., passive or iontophoretic.

Preferably, the pharmaceutical compositions are adapted to oral or parenteral (especially intravenous, intra-arterial or transdermal) administration. Intravenous and oral, first and foremost intravenous, administration is considered to be of particular importance. Preferably the zoledronic acid active ingredient is in the form of a parenteral, most preferably an intravenous form.

The particular mode of administration and the dosage may be selected by the attending physician taking into account the particulars of the patient, especially age, weight, life style, activity level, hormonal status, e.g., post-menopausal, and bone mineral density as appropriate. Most preferably, however, the zoledronic acid is administered intravenously.

The dosage of the zoledronic acid may depend on various factors, mode of administration, warm-blooded species, and/or sex, age, weight and individual condition of the warm-blooded animal.

Normally the dosage is such that a single dose of zoledronic acid or salt or hydrate thereof from 0.002-20.0 mg/kg, especially 0.01-10.0 mg/kg, is administered to a warm-blooded animal weighing approximately 75 kg. If desired, this dose may also be taken in several, optionally equal, partial doses. Doses of zoledronic acid or salts or hydrates thereof in the range from about 0.5 mg to about 20 mg, preferably from about 1 mg to about 10 mg, more preferably 5 mg, may be used for treatment of human patients.

Where the zoledronic acid or salt or hydrate thereof is given intravenously, the 5 mg dose is generally administered over a 15-minute period although shorter and longer periods are possible.

"mg/kg" means mg drug per kg body weight of the mammal - including man - to be treated.

In accordance with the present invention, the zoledronic acid is doses at intervals of once a year, (cf. co-pending International Patent Application WO 01/97788),

The dose mentioned above, either administered as a single dose (which is preferred) or in several partial doses, is administered once per year (understanding, of course, that it may not be exactly one year to date but rather at yearly check-ups).

Formulations in single dose unit form contain preferably from about 1 % to about 90%, and formulations not in single dose unit form contain preferably from about 0.1 % to about 20%, of the zoledronic acid active ingredient. Single dose unit forms, such as capsules, tablets or dragées contain, e.g., from about 1 mg to about 500 mg of the zoledronic acid active ingredient.

Pharmaceutical preparations for enteral and parenteral administration are, for example, those in dosage unit forms, such as dragées, tablets or capsules and also ampoules. They are prepared in a manner known *per se,* for example, by means of conventional mixing, granulating, confectioning, dissolving or lyophilizing processes.

For example, pharmaceutical preparations for oral administration can be obtained by combining the active ingredient with solid carriers, where appropriate granulating a resulting mixture, and processing the mixture or granulate, if desired or necessary after the addition of suitable adjuncts, into tablets or dragée cores. Suitable carriers are especially fillers, such as sugars, for example, lactose, saccharose, mannitol or sorbitol, cellulose preparations and/or calcium phosphates, for example, tricalcium phosphate or calcium hydrogen phosphate, and also binders, such as starch pastes, using, for example, corn, wheat, rice or potato starch, gelatin, tragacanth, methylcellulose and/or polyvinylpyrrolidone and, if desired, disintegrators, such as the above-mentioned starches, also carboxymethyl starch, crosslinked polyvinylpyrrolidone, agar or alginic acid or a salt thereof, such as sodium alginate. Adjuncts are especially flow-regulating agents and lubricants, for example, silicic acid, talc, stearic acid or salts thereof, such as magnesium or calcium stearate, and/or polyethylene glycol. Dragée cores are provided with suitable coatings that may be resistant to gastric juices, there being used, *inter alia,* concentrated sugar solutions that optionally contain gum arabic, talc, polyvinylpyrrolidone, polyethylene glycol and/or titanium dioxide, or lacquer solutions in suitable organic solvents or solvent mixtures or, to produce coatings that are resistant to gastric juices, solutions of suitable cellulose preparations, such as acetylcellulose phthalate or hydroxypropylmethylcellulose phthalate. Coloring substances or pigments may be added to the tablets or dragée coatings, for example for the purpose of identification or to indicate different doses of active ingredient.

Other orally administrable pharmaceutical preparations are dry-filled capsules made of gelatin, and also soft, sealed capsules made of gelatin and a plasticizer, such as glycerol or sorbitol. The dry-filled capsules may contain the active ingredient in the form of a granulate, for example, in admixture with fillers, such as lactose; binders, such as starches; and/or glidants, such as talc or magnesium stearate, and, where appropriate, stabilizers. In soft capsules, the active ingredient is preferably dissolved or suspended in suitable liquids, such as fatty oils, paraffin oil or liquid polyethylene glycols, it being possible also for stabilizers to be added.

Parenteral formulations are especially injectable fluids that are effective in various manners, such as intra-arterially, intramuscularly, intraperitoneally, intranasally, intradermally, subcutaneously or preferably intravenously. Such fluids are preferably isotonic aqueous solutions or suspensions which can be prepared before use, for example, from lyophilized preparations which contain the active ingredient alone or together with a pharmaceutically acceptable carrier. The pharmaceutical preparations may be sterilized and/or contain adjuncts, for example preservatives, stabilizers, wetting agents and/or emulsifiers, solubilizers, salts for regulating the osmotic pressure and/or buffers.

Suitable formulations for transdermal application include an effective amount of the zoledronic acid active ingredient with carrier. Advantageous carriers include absorbable pharmacologically acceptable solvents to assist passage through the skin of the host. Characteristically, transdermal devices are in the form of a bandage comprising a backing member, a reservoir containing the compound optionally with carriers, optionally a rate controlling barrier to deliver the active ingredient of the skin of the host at a controlled and predetermined rate over a prolonged period of time, and means to secure the device to the skin.

### EXAMPLES

### Example 1: Preparation of Capsules Containing Coated Pellets of Active Ingredient

| Core pellet: | | |
|---|---|---|
| | Active ingredient (ground) | 197.3 mg |
| | Microcrystalline cellulose | 52.7 mg |
| | (Avicel® PH 105) | |
| | | 250.0 mg |

| + Inner coating: | | |
|---|---|---|
| | Cellulose HP-M 603 | 10.0 mg |
| | Polyethylene glycol | 2.0 mg |
| | Talc | 8.0 mg |
| | | 270.0 mg |

| + Gastric juice-resistant outer coating: | | |
|---|---|---|
| | Eudragit^{®} L 30 D (solid) | 90.0 mg |
| | Triethyl citrate | 21.0 mg |
| | Antifoam^{®} AF | 2.0 mg |
| | Water | |
| | Talc | 7.0 mg |
| | | 390.0 mg |

A mixture of active ingredient with Avicel^{®} PH 105 is moistened with water and kneaded, extruded and formed into spheres. The dried pellets are then successively coated in the fluidized bed with an inner coating, consisting of cellulose HP-M 603, polyethylene glycol (PEG) 8000 and talc, and the aqueous gastric juice-resistant coat, consisting of Eudragit^{®} L 30 D, triethyl citrate and Antifoam^{®} AF. The coated pellets are powdered with talc and filled into capsules (capsule size 0) by means of a commercial capsule filling machine, for example, Höfliger and Karg.

### Example 2. Monolith Adhesive Transdermal System Containing 1-hydroxy-2-(imidazol-1-yl)-ethane-1,1-diphosphonic acid (zoledronic acid)

### Composition:

| | | |
|---|---|---|
| Polyisobutylene (PIB) 300 | | 5.0 g |
| | (Oppanol B1, BASF) | |
| PIB 35000 | | 3.0 g |
| | (Oppanol B10, BASF) | |
| PIB 1200000 | | 9.0 g |
| | (Oppanol B100, BASF) | |
| Hydrogenated hydrocarbon resin | | 43.0 g |
| | (Escorez 5320, Exxon) | |
| 1-dodecylazacycloheptan-2-one | | 20.0 g |
| | (Azone, Nelson Res., Irvine/CA) | |
| Active Ingredient | | 20.0 g |
| Total | | 100.0 g |

### Preparation:

The above components are together dissolved in 150 g of special boiling point petroleum fraction 100-125 by rolling on a roller gear bed. The solution is applied to a polyester film (Hostaphan, Kalle) by means of a spreading device using a 300 mm doctor blade, giving a coating of about 75 g/m². After drying (15 minutes at 60°C), a silicone-treated polyester film (thickness 75 mm, Laufenberg) is applied as the peel-off film. The finished systems are punched out in sizes in the wanted form of from 5-30 cm² using a punching tool. The complete systems are sealed individually in sachets of aluminized paper.

### Example 3: Vial Containing 1.0 mg Dry, Lyophilized 1-hydroxy-2-(imidazol-1-yl)ethane-1,1-diphosphonic acid (mixed sodium salts thereof)

After dilution with 1 mL of water, a solution (concentration 1 mg/mL) for i.v. infusion is obtained.

### Composition:

| | | |
|---|---|---|
| Active Ingredient (free diphosphonic acid) | | 1.0 mg |
| Mannitol | | 46.0 mg |
| Trisodium Citrate x 2 H₂O | ca. | 3.0 mg |
| Water | | 1 mL |
| Water for Injection | | 1 mL |

In 1 mL of water, the active ingredient is titrated with trisodium citrate x 2 H₂O to pH 6.0. Then, the mannitol is added and the solution is lyophilized and the lyophilisate filled into a vial.

### Example 4: Ampoule Containing Active Ingredient Dissolved in Water

The solution (concentration 3 mg/mL) is for i.v. infusion after dilution.

### Composition:

| | |
|---|---|
| Active ingredient | 19.73 mg |
| (≙ 5.0 mg of anhydrous active ingredient) | |
| Mannitol | 250 mg |
| Water for injection | 5 mL |

### Example 5: Treatment of Patients

"A Multinational, multicenter, double-blind, randomized, placebo-controlled, parallel-group study assessing the efficacy of intravenous zoledronic acid in preventing subsequent osteoporotic fractures after a hip fracture" is undertaken.

In the trial, zoledronic acid is used in the form of the free acid.

### Study objectives

The primary objective is to demonstrate, that zoledronic acid, administered annually (5 mg i.v. over 15 minutes) plus a loading dose of 75,000 - 125,000 units of vitamin D2 IM or orally once or 50,000 - 75,000 units of vitamin D3 IM or orally once and a maintenance dose of 800 - 1200 IU of vitamin D p.o. daily and elemental calcium (1000 - 1500 mg p.o. daily in a divided dose) to men and women after surgical repair of low-trauma hip fracture will significantly reduce the rate of clinical fractures, defined as all subsequent osteoporotic fractures, compared to a loading dose of 75,000 - 125,000 units of vitamin D2 IM or orally once or 50,000 - 75,000 units of vitamin D3 IM or orally once and a maintenance dose of 800 - 1200 IU of vitamin D p.o. daily and elemental calcium (1000 - 1500 mg p.o. daily in a divided dose) alone.

Secondary objective is to:
1. Demonstrate an increase in Bone Mineral Density (BMD) in the total hip and femoral neck, using dual X-ray absorptiometry (DXA), of the non-fracture hip at:
   - 12 months compared to randomization (Baseline)
   - 24 months compared to randomization (Baseline)
   - After 24 months, an annual BMD will be compared to randomization (Baseline)

Total hip BMD and femoral neck BMD will be collected by DXA at each investigator site and reported on CRF page at randomization visit and every 12 month visit thereafter. Percentage change from baseline of total hip BMD and femoral neck BMD will be computed for each of those patients, and used for the analysis.

### Overall study design

This will be a multicenter, randomized, double-blind, placebo-controlled, parallel group trial in men and women. Patients undergoing recent surgical repair of a low-trauma hip fracture will be eligible for enrollment. There will be at least 3 study contacts with patients after randomization within the first 24 months. Patients will have additional visits annually until 211 patients have reached the primary endpoint. The final visit for patients will be contingent on when this endpoint is achieved. Once the endpoint is achieved all patients will need to come in for a final visit. The final visit will be no less than 30 days from the patient's last dose of study medication or no more than 90 days after the day 211 patients have reached the primary endpoint. All patients signing informed consent at screening will receive a loading dose of 75,000 - 125,000 units of vitamin D2 or 50,000 - 75,000 units of vitamin D3 IM or orally and then begin a maintenance dose of 800 - 1200 IU of vitamin D p.o daily and elemental calcium (1000 - 1500 mg p.o. daily in a divided dose). Subjects will continue their daily maintenance dose of 800 - 1200 IU of vitamin D and elemental calcium (1000 - 1500 mg p.o. daily in a divided dose) at least 14 days prior to receiving study drug (zoledronic acid 5 mg IV over 15 minutes or placebo). Subjects may receive their study drug infusion at any time between day 14 post vitamin D2 or D3 administration and 90 days after surgical repair of their low trauma hip fracture. In special cases where a patient is to be randomized in less than 14 days a 25-OH vitamin D level result must be available and greater than or equal to 15 ng/ml, so the patient can be dosed. The patients will receive study drug (zoledronic acid 5 mg IV over 15 minutes or placebo) every 12 months until 211 patients have reached the primary endpoint. The study drug will be prepared at the site and zoledronic acid or placebo will be dispensed according to randomization received through an Interactive Voice Response System (IVRS).

End points rather than patients will power the study. The Steering Committee, though blinded to treatment group, may recommend changing target enrollment or extending follow-up time based on the event rate observed during interim event rate analyses. Approximately 115 sites will be recruited: approximately 40 in the United States, approximately 10 in Canada, and approximately 65 in the rest of the world to enroll approximately 1714 patients which are estimated to achieve 211 clinical fracture events.

The randomization will be blocked within sites so that near balance in the allocation of drug and placebo is achieved. Patients will be contacted by phone every 3 months starting after visit 3. Subjects will be contacted every 3 months between annual visits. Self-reports of fractures, will be recorded at each study contact and at any time identified during the study.

Hip BMD will be measured using dual X-ray absorptiometry (DXA) of the non-fracture hip at randomization and every 12 months during the study. If the site is unable to measure hip BMD of non-fracture hip then a lumbar spine BMD will be measured for safety only.

After the second dose of study medication the patients will be returning to the clinic once a year for their annual infusion. It is mandatory to follow-up with patients every three months with a phone call during the entire study. Phone contacts are a good way to solicit information regarding maintain patient retention. This phone contact is a way to assess for new fractures, SAEs and AEs.

Concomitant medications and co-morbid illnesses will be recorded at each study visit and vital signs will be measured at randomization. Height and weight will be measured at, randomization, 12 months and every annual visit, thereafter. A chemistry panel will be measured at randomization, 12 months and every 12 months, thereafter. Calculated creatinine clearance will also be determined within 4 weeks of randomization, 12 month visit and every 12 months, thereafter. The calculated creatinine clearance must be known within 4 weeks prior to giving study drug to the patient. A CBC will be performed at visit 1 for baseline safety assessment of eligibility.

Chest and hip X-rays will be collected at screening. Hip X-ray of the incident hip will be performed at 6 months. Hip fracture healing will be assessed using clinical signs and symptom (persistent pain and/or inability to bear weight on index hip) at randomization, 6 and 12 months following the index fracture repair. All subjects reporting these signs or symptoms will have hip radiographs reviewed by the CEC.

In countries, where applicable, data on resource utilization use will be collected at screening, randomization, 6, 12 and 24 months. EQ-5D will be administered at randomization, 6, 12, and 24 months.

### Patient population

The study population will consist of men and women aged 50 years and older who have suffered a recent low-trauma, acute hip fracture and were ambulatory prior to the fracture. Patients admitted to orthopedic surgical or medical services, extended care or rehabilitation facilities and seen in clinic will be identified. Eligible patients will be ascertained from hospital admission logs, or operating room logs, extended care facility/rehabilitation logs and clinic schedules. At this time, the purpose of the study will be explained to the patient and, if necessary, their surrogates. If the patient/surrogate agrees to participate, the informed consent form will be signed and a chemistry panel, calculated creatinine clearance and a CBC will be performed. Patients will be instructed to take the vitamin D loading dose, vitamin D and elemental calcium supplements daily. After 14 days, if the results of laboratory tests are determined to comply with the inclusion/exclusion criteria, patients will be randomized to a treatment group and will receive their first dose of study drug at visit 2 (randomization). A supply of vitamin D and elemental calcium supplements will be provided. An estimated total of 1714 patients will be enrolled into this trial (to provide 211 fracture events

Because of the high incidence of delirium after hip fracture surgery, the patient's capacity to provide informed consent will be carefully assessed. Study personnel obtaining consent will review the patient's chart for documentation of intermittent confusion, and seek information about cognitive status from the patient's nurse and/or primary physician. The Confusion Assessment method (CAM) (or other locally adapted usual practice for delirium assessment) will be used to screen for delirium. Those patients judged to lack capacity for informed consent by study personnel or clinical staff will be given a brief explanation of the study and asked for assent. If this is given, full informed consent will be sought from the patient's legally authorized representative. The legal representative will be determined according to the state or national laws governing the study site and in accordance with ICH guidelines.

The investigator or designee must explain to each patient or legally authorized representative the nature of the study, its purpose, the procedures involved, the expected duration, the potential risks and benefits involved and any discomfort it may entail. Each patient must be informed that participation in the study is voluntary, that he/she may withdraw from the study at any time and that withdrawal of consent will not affect subsequent medical treatments or relationships with treating physicians. Informed consent will be given by means of a standard written statement, written in non-technical language. The patient should read and consider the statement before signing and dating it and will be given a copy of the signed document. If written consent is not possible, oral consent can be obtained if witnessed by one or more persons not involved in the study, and the reason why the patient was unable to sign the form must be documented. No patient can enter the study before informed consent has been obtained.

### Inclusion criteria

1. Male or female patient aged great er than or equal to 50 years
2. Patient may be randomized up to 90 days post-surgical repair of a low-trauma hip fracture
3. Patient was ambulatory with or without assistive device prior to the hip fracture
4. Patient must have intact both lower appendages (legs), not an amputee

### Exclusion criteria

1. Subject or their physician prefers to use an oral bisphosphonate (N.B. oral bisphosphonate safety and efficacy has not been studied in this population)
2. Treatment with any investigational drug within the past 30 days prior to randomization
3. Previous history of allergic reaction or hypersensitivity to bisphosphonates
4. History of uveitis or iritis, except when secondary to trauma, and this must have resolved greater than 2 years prior to randomization.
5. Calculated Creatinine Clearance less than or equal to 30.0 ml/min
6. Serum calcium greater than 2.75 mmoUL (11.0 mg/dL)
7. Serum alkaline phosphatase, greater than 2.5 X ULN
8. Hypocalcemia (serum corrected calcium less than 8 mg/dl or 2.0 mmol/L at screening and/or randomization)
9. Primary hyperparathyroidism, hypoparathyroidism, Osteogenesis imperfecta, Paget's disease, or any other metabolic bone disease, except osteoporosis
10. Cancer Exclusion:
   - Patients with a new diagnosis or active treatment for any malignancy less than or equal to 12 months prior to randomization.
   - Patients with known metastases (or by history)
   - Patients with the following *may* be included: basal cell or squamous cell carcinoma of the skin, colonic polyps with non-invasive malignancy which have been removed, Ductal Carcinoma in-situ (DCIS) that has been surgically removed, and Carcinoma in-situ (CIS) of the uterine cervix that has been surgically removed)
11. Previous major solid organ transplant recipient less than 2 years ago, prior to randomization or on a transplant waiting list
12. Any prior use of i.v. bisphosphonate within the last 2 years
13. Washout period for any oral bisphosphonate:
   2 years (if used for greater than 48 weeks)
   1 year (if used for greater than 8 weeks but less than 48 weeks)
   6 months (if used for greater than 2 weeks but less than or equal to 8 weeks)
14. Any prior use of PTH and PTH analogs for more than 1 week; if used for less than or equal to 1 week, wash out period for PTH and PTH analogs is 6 months. The reference point for the washout period should be the date of randomization
15. Any prior use of sodium fluoride treatment for osteoporosis
16. Any prior use of strontium (all formulations)
17. Hip fractures unlikely to be due to osteoporosis (traumatic fracture, malignant fracture, osteomyelitic fracture, hardware related fracture)
18. Serious disease and/or any clinically significant laboratory findings that in the opinion of the investigator could seriously affect the patients ability to participate in the study or that may limit life expectancy to less than 6 months
19. Other conditions/circumstances likely to lead to poor treatment adherence
20. Pregnant, lactating, or has the potential to become pregnant and does not agree to use an effective contraceptive method

### Interruption or discontinuation of treatment

Any use of bisphosphonates (other than study therapy), PTH and PTH analogs, fluoride, strontium, or anabolic steroids, except testosterone in the case of hormone replacement therapy in hypogonadal men, for more than two weeks during the study will be the cause for discontinuation of the patient from study treatment. However, all patients will remain in the trial for observation regardless of adherence to study medication.

Patients who have been unblinded will also be discontinued from study treatment.

It will be documented whether or not each patient completed the clinical study. If study treatment or observations are discontinued for a patient, the reason will be recorded. Reasons that a patient may discontinue participation in a clinical study are considered to constitute one of the following:
1. adverse event(s)
2. abnormal laboratory value(s)
3. abnormal test procedure result(s)
4. unsatisfactory therapeutic effect
5. subject's condition no longer requires study treatment
6. protocol violation
7. subject withdrew consent
8. lost to follow-up
9. administrative problems
10. death

The IVRS must also be called and the patient's discontinuation reported accordingly.

Follow-up for patients no longer on study medications will include fracture information, serious and non-serious AEs and all other regular study measurements (labs, DXA, X-rays). These patients will continue to be provided with calcium and vitamin D supplements. For patients who do not wish to continue with follow-up visits or withdraw consent, the final visit CRFs and procedures will be completed. However, the DXA and X-rays should not be performed if performed in the preceding 3 months, unless there is clinical cause to do so.

### Investigational therapy and reference therapy

Zoledronic acid or placebo (5.0 mg) will be administered within 90 days of surgical repair of hip fracture, at the 12 month study visit and annually until the study is completed, as a result of the required number of fractures have occurred and have been adjudicated. Zoledronic acid or placebo (physiologic 0.9% normal saline) is to be given intravenously to each patient as a slow infusion over 15 minutes. A patient's supply of vitamin D and elemental calcium will be provided to satisfy the patient's requirement until the next scheduled visit.

Patient specific double blinded drug kits will be prepared by Clinical Trial Services (CTS). Active drug kits will contain vials of zoledronic acid (5 mg in 5 ml of sterile water for injection) and 2 (10mL) physiologic (0.9%) normal saline for the flushing of the intravenous line. Placebo kits will contain identical placebo vials (excipients in 5 ml of sterile water) and 2 (10mL) physiologic (0.9%) normal saline for the flushing of the intravenous line. Bulk supplies of physiologic (0.9%) normal saline for a diluted total injection volume of 105 ml will be sent only to clinical sites in the United States and Canada, other countries will use their own supply. Bulk supplies of vitamin D2, vitamin D and elemental calcium will also be sent to clinical sites in the United States and Canada, other countries, as applicable, will use their own supply. The pharmacist or other qualified health professional will be responsible for the preparation of the i.v. zoledronic acid and i.v. placebo. The qualified person who prepares the drug to be infused must enter the appropriate drug preparation information requested on the sign off log for drug preparation. United States and Canadian sites will be provided with an adhesive label (to indicate that drug must be infused over 15 minutes) to be attached to each 105ml i.v. bag of drug (active or placebo) that is prepared. Documentation of trial-drug administration (time the infusion started and ended) and amount of infusion given will be captured for each patient in the CRF.

Zoledronic acid must not be stored above 30°C.

The study drug will be stored at room temperature in a locked area at each center until they are returned to Novartis or its designee at the end of the study. Diluted zoledronic acid infusion solutions are stable for at least 24 hours at 2-8°C. The cumulative time between dilution with infusion media, storage in a refrigerator and end of administration must not be longer than 24 hours. Ideally, diluted zoledronic acid solutions should be used immediately. However, if diluted zoledronic acid solutions cannot be used immediately, the solutions must be refrigerated at temperatures between 2-8°C. Before administration, the solution should be allowed to slowly warm to room temperature again.

Since Novartis will not supply the plastic i.v. infusion bag to countries outside of the United States and Canada, the study-drug solutions should be prepared in plastic syringes and tubings (polyvinylchloride, polyethtylene or polypropylene) and infused from a plastic bag or a glass bottle. Zoledronic acid or placebo is to be given intravenously to each patient as a slow infusion over 15 minutes. The appropriate volume of zoledronic acid is to be mixed with an appropriate volume of physiologic (0.9%) normal saline so that the total volume of infused solution is 105 ml.

Patients should be encouraged to have sufficient food and liquid intake, at dosing and for several days following the dose as no special dietary restrictions apply.

### Concomitant Medications:

Due to the age of the patient population studied, it is likely that many patients will have other significant medical problems requiring medications as therapy. Since many common medications affect bone metabolism, an accurate account of these medications must be documented during the period of the study. Patients will be asked about use of the medications below. The name of the drug should be recorded on the concomitant medication/therapy page in the CRF.

The following medications will not be allowed during the study:
- Bisphosphonates other than the study medication
- Sodium fluoride
- PTH and PTH analogs
- Anabolic steroids except testosterone in the case of hormone replacement therapy in hypogonadal men
- Strontium
- Any investigational therapy other than the study medication

At each patient contact, patients will be queried on the use of concomitant medications. Since this trial will compare usual medical care for patients with hip fractures against zoledronic acid, patients will be allowed to receive all approved therapies for osteoporosis except those mentioned above. Since less than 10% of hip fracture patients receive any therapy for their osteoporosis, the use of concomitant osteoporosis therapies (calcitonin, SERMs (e.g. raloxifene), hormone replacement therapy (HRT), tibolone, DHEA(s), ipriflavone, and testosterone, as hormone replacement in the case of hypogonadal men), with the exception of those listed above, is not anticipated to affect the outcome of this trial.

### Primary endpoint

Clinically evident fractures occurring in the follow-up period are the primary endpoint. Facial, skull and digital fractures are not associated with osteoporosis and will not qualify as an outcome. The site study coordinator will obtain copies of radiology reports or physician's chart documentation of X-ray results for non-vertebral fractures. These will be submitted to the Clinical Endpoint Committee (CEC) within 1 week of the patient's report of the event. Radiographs will also be requested for some fractures that require further confirmation.

The diagnosis of clinically evident vertebral fracture will require the following: 1) acute onset or worsening back pain in a localized area of the spine as triggered in the CRF, and 2) PA and lateral lumbar spine films (obtained during routine clinical care) showing one or more grade of vertebral height loss by the semi-quantitative technique of Genant et al in comparison with baseline X-rays. For men, a modification of the Genant criteria will be used in order to improve specificity for vertebral fracture. If no baseline films are available, an incident fracture will be defined as one or more vertebrae with grade 2 or higher deformity. If modalities other than plain film are used to diagnose the vertebral fracture, an incident fracture is defined as a significant deformity in a vertebrae with no greater than grade 1 deformity at baseline.

If a patient suffers more than one fracture after hip fracture, the first such event will be counted as the primary endpoint. If a patient suffers more than one fracture at the same time, the most clinically serious will be counted as the primary endpoint in the following hierarchy: hip, long bone, vertebral, wrist, other.

Both traumatic and minimally traumatic fractures will be considered endpoints. New fractures associated with orthopedic hardware are of importance and will also be considered primary endpoints. Fractures judged by the CEC to be due to metastatic cancer, osteomyelitis or high energy trauma (eg. motor vehicle collision or falls from greater than standing height) will not be considered endpoints.

### Secondary endpoints

1. Demonstrate an increase in Bone Mineral Density (BMD) of the total hip and femoral neck BMD, using dual X-ray absorptiometry (DXA) of the non-fracture hip at:
   - 12 months compared to randomization (Baseline)
   - 24 months compared to randomization (Baseline)
   - After 24 months, an annual BMD will be compared to randomization (Baseline)

Total hip BMD and femoral neck BMD will be measured by DXA at each investigator site and reported on CRF page at randomization visit and every 12 month visit thereafter. Percentage change from baseline of total hip BMD and femoral neck BMD will be computed for each of those patients, and used for the analysis.

### Statistical methods

The study is designed to show superiority of zoledronic acid compared to placebo in reducing clinical fracture rate after surgical repair of low-trauma hip fracture. The primary endpoint is "time to event" (time from randomization to first clinical fracture), and log-rank test will be used in the primary analysis. The primary analysis population is intent-to-treat. This trial will continue until 211 patients reached primary endpoint.

It is planned that the data from all centers that participate in this protocol will be combined, so that an adequate number of subjects will be available for analysis.

The specifications described are those envisaged at the time of planning the trial.

### Populations

### Safety population

All subjects randomized to treatment, and who have been exposed to study drug.

### Intent-to-treat population (ITT)

Intent-to-treat analysis data set will include all randomized subjects.

### Per-protocol population (PP)

Per-protocol analysis data set will include all subjects without any major protocol violation. The protocol violations will be identified before unblinding.

### Background and demographic characteristics

Summary statistics for background and demographic variables will be provided by treatment groups. In addition, the randomization comparability between treatment groups will be evaluated for the background and demographic variables. Categorical variables will be evaluated using Fisher's exact test, and the continuous variables will be evaluated using a one-way analysis of variance model.

Note these tests of comparability are performed for descriptive purpose only, and will not be considered to define any formal basis for determining factors, which should be included in statistical analysis models. However, these tests can be used as extra information in interpreting the statistical analyses performed on the primary endpoint.

### Study medication

Summary statistics for the number of infusions will be provided by treatment groups.

### Concomitant therapy

Summary statistics will be provided for the concomitant therapy both prior to and after start of study drug administration. Fisher's exact test will be used to test the comparability between treatment groups with respect to proportion of subjects who take concomitant osteoporosis medications and NSAIDs/ACE inhibitors.

### Efficacy evaluation

The analysis of primary efficacy variable will be performed on the intent-to-treat and per-protocol populations. The primary analysis population is intent-to-treat population. The analysis of secondary efficacy variables will be performed on intent-to-treat population. In addition, adjustment for multiple comparisons will not be made for any of the secondary efficacy variables.

### Primary efficacy analysis

The primary efficacy endpoint is time to first clinical fracture. Between-treatment differences will be evaluated using a log-rank test to compare the time to clinical fracture between the two treatment groups. The Kaplan-Meier estimates of the time to clinical fracture for each treatment will be plotted. The Kaplan-Meier estimates of incidence of clinical fractures at the end of study will be presented.

The calculation of time to clinical fracture event will be determined based on the following methods:
- If a clinical fracture has occurred, the time to clinical fracture event will be computed from the date of first infusion to the detection date of fracture.
- If a clinical fracture has not occurred and the subject completes the study, the censoring time will be computed from the date of first infusion to the date of the last study visit.
- If a subject dies without a clinical fracture, then the censoring time will be computed from the date of first infusion to the date of death.
- If a subject is lost to follow-up without a clinical fracture, then the censoring time will be computed from the date of first infusion to the last available visit date in the final database.

Since the clinical fracture rate in some centers/countries can be very small or even zero, centers/countries will be pooled into regions to assess any geographical differences that may exist between treatments. A pooling scheme will be decided before unblinding. Tabular and/or graphical methods will be used to assess treatment-by-region interaction as appropriate (secondary analysis on primary endpoint).

Since one subject can have more than one clinical fracture (multiple events data), as an exploratory analysis, the Anderson-Gill type approach (18) will be used to explore the between-treatment differences with respect to all clinical fracture rates.

### Secondary efficacy analysis

The secondary efficacy endpoints are the percent change relative to *randomization* of total hip BMD and femoral neck BMD at month 12, 24 and every 12 months thereafter.

Percent change from *randomization* at each visit will be analyzed for BMD efficacy variables. Between-treatment differences will be evaluated using a two-way analysis of variance (ANOVA) model with treatment and center as explanatory variables.

A positive outcome is indicated by both the 12-month and 24-month results showing a significant reduction in secondary osteoporotic fractures in patients who have recently undergone surgical repair of a hip fracture when they take zoledronic acid once per year in combination with vitamin D and calcium therapy vs. those patients taking vitamin D and calcium therapy alone.

## Claims

1. Use of zoledronic acid or a pharmaceutically acceptable salt or hydrate thereof in the preparation of a medicament for the treatment of osteoporosis post hip fracture **characterised in that** the treatment is to prevent or reduce subsequent osteoporotic skeletal fractures in a patient in need of such treatment, said patient having undergone repair of a hip fracture within the past 90 days, and wherein the medicament is administered once per year.

2. A use according to claim 1 wherein administration of the medicament is within 60 days of the repair of the hip fracture.

3. A use according to claim 1 wherein administration of the medicament is within 42 days of the repair of the hip fracture.

4. A use according to claim 1 wherein administration of the medicament is within 1-7 days of the repair of the hip fracture.

5. A use according to any one of the preceding claims wherein the medicament is for intravenous administration.

6. A use according to any one of the preceding claims wherein the medicament is in unit dose form and the effective amount of zoledronic acid is 5 mg.

7. A use according to any one of the preceding claims wherein the zoledronic acid is the free acid.

8. A use according to claim 7, wherein the effective amount of the free acid is 5 mg for administration intravenously over a 15-minute period.

9. A use according to claim 1 wherein said patient is administered vitamin D2 prior to administration of the zoledronic acid.

10. A use according to claim 9, wherein the vitamin D2 is administered at least 14 days prior to administration of the zoledronic acid.

11. A use according to claim 1 wherein the patient is administered a vitamin D supplement on a daily basis.

12. A use according to claim 1 wherein the patient is administered a calcium supplement on a daily basis.

## Patentansprüche

1. Verwendung von Zoledronsäure oder eines pharmazeutisch akzeptablen Salzes oder Hydrats hiervon bei der Herstellung eines Medikaments zur Behandlung von Osteoporose nach einer hüftgelenksnahen Femurfraktur (proximale Oberschenkelfraktur), **dadurch gekennzeichnet, dass** die Behandlung dazu dient, osteoporotische skelettale Folgefrakturen bei einem Patienten, der eine derartige Behandlung benötigt, zu verhindern oder zu verringern, wobei der Patient innerhalb der vergangenen 90 Tage einer Wiederherstellung (Instandsetzung, Reparatur) einer hüftgelenksnahen Femurfraktur unterzogen war, und wobei das Medikament einmal pro Jahr verabreich wird.

2. Verwendung gemäß Anspruch 1, wobei die Verabreichung des Medikaments innerhalb von 60 Tagen nach der Wiederherstellung der hüftgelenksnahem Femurfraktur erfolgt.

3. Verwendung gemäß Anspruch 1, wobei die Verabreichung des Medikaments innerhalb von 42 Tagen nach der Wiederherstellung der hüftgelenksnahem Femurfraktur erfolgt.

4. Verwendung gemäß Anspruch 1, wobei die Verabreichung des Medikaments innerhalb von 1-7 Tagen nach der Wiederherstellung der hüftgelenksnahem Femurfraktur erfolgt.

5. Verwendung nach einem der vorhergehenden Ansprüche, wobei das Medikament für eine intravenöse Verabreichung geeignet ist.

6. Verwendung nach einem der vorhergehenden Ansprüche, wobei das Medikament in Einheitsdosierungsform vorliegt und die wirksame Menge von Zoledronsäure 5 mg beträgt.

7. Verwendung nach einem der vorhergehenden Ansprüche, wobei es sich bei der Zoledronsäure um die freie Säure handelt.

8. Verwendung gemäß Anspruch 7, wobei die wirksame Menge der freien Säure 5 mg für die intravenöse Verabreichung über einen 15-minütigen Zeitraum beträgt.

9. Verwendung nach Anspruch 1, wobei dem Patienten Vitamin D2 vor einer Verabreichung der Zoledronsäure verabreicht wird.

10. Verwendung nach Anspruch 9, wobei das Vitamin D2 mindestens 14 Tage vor einer Verabreichung der Zoledronsäure verabreicht wird.

11. Verwendung nach Anspruch 1, wobei dem Patienten eine Vitamin D-Ergänzung auf einer täglichen Basis verabreicht wird.

12. Verwendung nach Anspruch 1, wobei dem Patienten eine Calciumergänzung auf einer täglichen Basis verabreicht wird.

## Revendications

1. Utilisation de l'acide zolédronique ou de l'un de ses sels ou hydrates acceptables sur le plan pharmaceutique, dans la préparation d'un médicament destiné au traitement de l'ostéoporose après une fracture de la hanche, **caractérisée en ce que** le traitement consiste à prévenir ou à réduire les fractures squelettiques ostéoporotiques ultérieures chez un patient ayant besoin d'un tel traitement, ledit patient ayant subi une réparation d'une fracture de la hanche dans les 90 jours qui précèdent, et où le médicament est administré une fois par an.

2. Utilisation selon la revendication 1, où l'administration du médicament se fait dans les 60 jours après la réparation de la fracture de la hanche.

3. Utilisation selon la revendication 1, où l'administration du médicament se fait dans les 42 jours après la réparation de la fracture de la hanche.

4. Utilisation selon la revendication 1, où l'administration du médicament se fait 1-7 jours après la réparation de la fracture de la hanche.

5. Utilisation selon l'une quelconque des revendications précédentes, où le médicament est destiné à l'administration intraveineuse.

6. Utilisation selon l'une quelconque des revendications précédentes, où le médicament est sous forme de dose unitaire et la quantité efficace d'acide zolédronique est de 5 mg.

7. Utilisation selon l'une quelconque des revendications précédentes, où l'acide zolédronique est l'acide libre.

8. Utilisation selon la revendication 7, où la quantité efficace de l'acide libre est de 5 mg pour une administration intraveineuse sur une période de 15 minutes.

9. Utilisation selon la revendication 1, où de la vitamine D2 est administrée audit patient avant l'administration de l'acide zolédronique.

10. Utilisation selon la revendication 9, où la vitamine D2 est administrée au moins 14 jours avant l'administration de l'acide zolédronique.

11. Utilisation selon la revendication 1, où un supplément de vitamine D est administré au patient sur une base journalière.

12. Utilisation selon la revendication 1, où un supplément de calcium est administré au patient sur une base journalière.
